# EUROPEAN PATENT APPLICATION

(11) **EP 1 132 481 A1**
(43) Date of publication of application: **12.09.2001**
(21) Application number: 00870041.1
(22) Date of filing: 10.03.2000
(51) Int. Cl.: C12Q 1/04, C12Q 1/34, C12Q 1/44

(54) **Detection of Aspergillus fumigatus**

(71) Applicant: UNIVERSITEIT GENT, 9000 Gent (BE)
(72) Inventor: Nelis, Hans J., 9667 Horebeke (BE); Bauters, Tiene G. M., 9080 Lochristi (BE)
(74) Representative: De Clercq, Ann

(57) **Abstract**

The present invention is based on the finding that micro-organisms can be detected and identified on the basis of a specific enzymatic activity present in the micro-organism to be detected. Methods and kits are described for the detection of micro-organisms using an arabinopyranoside substrate and more particularly methods and kits are described for the specific , sensitive and rapid detection *of Aspergillus fumigatus.*

## Description

### Field of the invention

The present invention relates to the field of the specific and selective detection and identification of micro-organisms based on a specific enzymatic activity present in the micro-organism to be detected. More particularly, the arabinopyranoside substrate cleaving activity present in specific micro-organisms is envisaged.

### Background of the invention

Invasive aspergillosis, mainly caused by *Aspergillus fumigatus* is a life-threatening condition that requires prompt antifungal therapy (Fridkin and Jarvis, 1996; Latgé, 1999). However, a rapid and accurate diagnosis of aspergillosis is still hampered by shortcomings in the currently available methodology. Current research is mostly concerned with the development of non-culture based molecular and immunological methods such as PCR and ELISA methods (Latgé, 1999; Paugam et al., 1998; Richardson and Warnock, 1997; Richardson, 1998; Van Burick et al., 1998; Verweij et al., 1995). However, apart from direct microscopic examination of samples, the isolation of the organism on an agar plate followed by microscopic inspection of the growth remains the backbone of the laboratory diagnosis in daily routine (Richardson and Warnock, 1997; Richardson, 1998). This type of procedure may take up to five days and other mold species, e.g. *Fusarium* and *Scediosporum apiospermum (Peudallescheria boydii*) may exhibit some of the morphological characteristics of *Aspergillus* sp. (Richardson and Warnock, 1997). False negative cultures of blood and broncho-alveolar lavage fluid are common (Latgé, 1999; Richardson, 1998; Van Burick et al., 1998).

### Summary of the invention

It is the aim of the present invention to provide a method to detect micro-organisms in a biological sample with an enhanced specificity and sensitivity for the micro-organism to be detected.

It is a further aim of the invention to provide a method for the rapid and specific identification of *Aspergillus fumigatus* in biological samples.

It is also an aim of the invention to provide a selective enriched growth medium for fungi.

### Detailed description of the invention

In a first embodiment the present invention relates to a method for detecting micro-organisms in a biological sample comprising testing for an arabinopyranoside substrate cleaving activity of said micro-organisms.

The expression "arabinopyranoside substrate cleaving activity" refers to an activity cleaving the glycosidic bond between the arabinopyranose and its aglycone in said arabinopyranoside substrate. An alternative for the expression "arabinopyranoside substrate cleaving activity" is the term "arabinopyranosidase". Examples of arabinopyranoside substrates are for instance, but not limited to the following: 4-methylumbelliferyl-α-L-arabinopyranoside, 4-trifluoromethylumbelliferyl-α-L-arabinopyranoside, o-nitrophenyl-α-L-arabinopyranoside, p-nitrophenyl-α-L-arabinopyranoside, indoxyl-α-L-arabinopyranoside and its substituted derivatives such as 5-bromo-4-chloro-3-indolyl-α-L-arabinopyranoside, 5-bromo-6-chloro-3-indolyl-α-L-arabinopyranoside, 6-chloro-3-indolyl-α-L-arabinopyranoside, N-methylindolyl-α-L-arabinopyranoside, chlorophenol red α-L-arabinopyranoside, resorufin-α-L-arabinopyranoside, and 3-{4-methoxyspiro [1,2-dioxetane-3,2'-tricyclo (3.3.1.1^{3.7})decan]-4-yl}-fenyl-α-L-arabinopyranoside and its chlorosubstituted derivatives.

The present inventors were able for the first time to demonstrate a specific arabinopyranoside substrate cleaving activity in *Aspergillus fumigatus* which was not found in other fungi nor in other *Aspergillus spp.* tested.

Therefore, in a preferred embodiment, the present invention relates to a method for detecting *Aspergillus fumigatus* in a biological sample comprising testing for an arabinopyranoside substrate cleaving activity.

The term "biological sample" refers to any liquid or liquefied sample comprising bronchoalveolar liquid, blood, CSF, serum, urine, oropharyngeal exudate and swabs, tube culture, nose swabs, ear swabs and sputum.

In a further embodiment, the present invention relates to a method as described above wherein said testing of arabinopyranoside substrate cleaving activity comprises the use of a fluorogenic, chromogenic or chemiluminogenic substrate.

The term fluorogenic substrate refers to a compound which itself is non-fluorescent but which contains a structural part, i.e. the so-called fluorescent product, that emits light when liberated from the parent compound and photochemically excited.

The term chromogenic substrate refers to a compound which itself is not colored but which contains a structural part, the so-called colored product, that is or becomes colored when liberated from the parent compound.

The term chemiluminogenic substrate refers to a compound which itself is not chemiluminescent but which contains a structural part, i.e. the so-called chemiluminescent product, that emits light when liberated from the parent compound and chemically excited.

Modification of compounds by fluorogenic, chromogenic and chemiluminogenic substituents is well known in the art (Bronstein et al., 1989; Ferguson, 1994; Koller, 1989; Manafi, 1991).

In a preferred embodiment, the present invention relates to the use of 4-methylumbelliferyl-α-L-arabinopyranoside, which is a fluorogenic substrate. The arabinopyranoside substrate cleaving activity of *Aspergillus fumigatus* by which the glycosidic bond between the arabinopyranose and 4-methylumbelliferone is cleaved, results in the formation of blue fluorescent microcolonies which can be visualised under a 366 nm UV lamp.

According to another embodiment, the present invention relates to a method for the detection of micro-organisms in a biological sample comprising at least the following steps:
a) concentrating the micro-organisms in said biological sample on a membrane filter,
b) incubating the membrane filter of step a) on a growth medium to form microcolonies,
c) removing said membrane filter from the growth medium,
d) contacting said membrane filter with a solution comprising an arabinopyranoside substrate possibly supplemented with a membrane permeabilizer,
e) possibly amplifying the signal by spraying said membrane filter with sodium hydroxide or another light amplifying material,
f) identifying the presence of fluorescent, colored or light-emitting microcolonies on said membrane filter.

The term "membrane permeabilizer" refers to any compound capable of disrupting the cytoplasmic membrane of the micro-organism in order to facilitate the uptake of chemicals.

The term "light amplifying material" refers to a chemical that intensifies light emission in fluorescence or chemiluminescence.

According to a preferred embodiment, the membrane permeabilizer used in the methods of the invention is digitonin. Other possible membrane permeabilizers are for instance toluene, amphotericin B, sodium lauryl sarcosinate, chloroform, dimethylsulfoxide, lysolecithin and higher alcohols, e.g. amyl alcohol.

In a preferred embodiment, the invention relates to the method as described above for the detection and identification of *Aspergillus fumigatus* using 4-methylumbelliferyl-α-L-arabinopyranoside as a substrate for demonstrating the arabinopyranoside substrate cleaving activity of said micro-organism.

It should be understood that the arabinopyranoside substrate could also be added to the growth medium. As such, the invention also relates to a method for the detection of micro-organisms in a biological sample comprising at least the following steps:
a) concentrating the micro-organisms in said biological sample on a membrane filter,
b) incubating the membrane filter of step a) on a growth medium to form microcolonies, said growth medium containing an arabinopyranoside substrate.
c) identifying the presence of arabinosidase positive microcolonies on said membrane filter.

In a preferred embodiment, the membrane filter according to any of the methods of the invention is a nylon membrane filter.

In another preferred embodiment, the growth medium for use in any of the methods of the invention is Sabouraud glucose agar further comprising growth inhibitors for bacteria such as ticarcillin and clavulanic acid and further enriched by the addition of a mixture of growth factors, for example pyridoxine, thiamine, nicotinic acid, riboflavine, panthothenate, para-aminobenzoic acid (PABA) and inositol.

The growth medium as described above has been optimized by the inventors to promote better growth of fungi such as *Aspergillus fumigatus* and as such this enriched growth medium also forms part of the present invention.

In a preferred embodiment, the incubation step b) in any method of the invention may be performed at 45°C. This higher incubation temperature further increases the selectivity of the method(s) of detecting the presence of *Aspergillus fumigatus.*

In another embodiment, the present invention relates to a kit for diagnosing the presence of a micro-organism in a biological sample comprising an arabinopyranoside substrate.

In a preferred embodiment, this kit comprises at least the following components:
a) a membrane filter,
b) a growth medium for the micro-organism(s) to be detected, and
c) a fluorogenic, chromogenic or chemiluminogenic arabinopyranosidase substrate for testing the arabinopyranoside substrate cleaving activity,
d) and possibly also sodium hydroxide or another light amplifying material for spraying the membrane filter to amplify the signal.

The membrane filter referred to in a) preferably is a nylon membrane filter.

According to the invention, the growth medium comprised in the kit preferably is a growth medium as defined in claim 7.

In another preferred embodiment, the kit for diagnosing the presence of a micro-organism in a biological sample comprising an arabinopyranoside substrate may further include an inducer for the arabinopyranoside substrate cleaving activity and/or a membrane permeabilizer.

The term "inducer" refers to molecules that promote the biosynthesis of an enzyme by enhancing the expression of the genes involved.

The invention, now being generally described, will be more readily understood by reference to the following examples, which are included merely for purposes of illustration of certain aspects and embodiments of the present invention and are not intended to limit the invention. All of the references mentioned herein are incorporated by reference.

### Brief description of Tables

***Table 1:*** Cleavage of 4- methylumbelliferyl-glucosides and esters by medically important fungi.

***Table 2:*** Frequency table representing numbers of identified species comparing the arabinosidase based method and a conventional identification method.

### EXAMPLES

### Example 1: Materials

### Fungal strains for method development.

A total of 134 *Aspergillus* and 30 *non-Aspergillus* spp. laboratory strains were used for the method development. These included *A*. *fumigatus* (n = 48), *A. flavus* (n = 29), *A. niger* (n = 22), *A*. *clavatus* (n = 4), *A*. *nidulans (n =* 13), *A. terreus* (n = 12), *A. versicolor* (n = 6), *Fusarium oxysporum* (n = 4), *Fusarium solani* (n = 4), *Rhizopus oryzae* (n = *4), Rhizopus microsporus* (n = 3), *Absidia corymbifera* (n = 2), *Rhizomucor pusillus* (n *= 4), Penicillium marneffei* (n = 5), and *Pseudallescheria boydii (n =* 4). The strains were obtained from the Mycothèque de l'Université Catholique de Louvain-La-Neuve (MUCL) (Louvain-La-Neuve, Belgium) *(A. fumigatus* 978,15821; *A. flavus* 1032,19007; *A*. *niger* 13608, 19001, 30113) and from the Institute of Hygiene and Epidemiology, division Mycology (IHEM) (Brussels, Belgium) *(A. fumigatus* 1995, 1997*-2001, 2003,* 2494, 2943, 2945, 2952, 3007, 3125, 3131, 3242, 3768, 4182-4189; *A. flavus* 306, 627, 2262, 2465, 2647, 2700, 3018, 3719, 3790, 4390, 5094, 5285, 5669, 5675, 5721, 5730, 5901-5904, 5906-5908, 6741, 9407, 9408; *A. niger* 2312, 2864, 2951, 3019, 3415, 3766, 3797, 4023, 4461, 4781, 5296, 5788, 6147, 6350, 6727, 9673, 9709, 14389; *A. clavatus* 5138, 6078, 7944, 9776; *A. nidulans* 1502, 1503, 1548, 2059, 3563, 3793, 4190, 5137, 5231, 5589, 6365, 9353, 9679; *A. terreus* 307, 349, 1945, 2499, 3280, 4395, 5677, 5918, 6640, 6946, 7940; *A. versicolor 2157,* 2158, 2646, 2916, 2983, 3202; *F. oxysporum* 9571, 3545, 14513; *F. solani* 591, 4831, 5601, 6743; *R. oryzae* 5215, 5233, 6016, 6017; *R. microsporus* 5208, 5234, 7978; *A. corymbifera* 3106, 5092; *R. pusillus* 1387, 2020, 4897, 10343; *P. marneffei* 3272, 4176, 6051-6053, *P. boydii* 1055, 3724, 3725, 3747). The remaining strains were clinical isolates originating from the department of Clinical Biology, Bacteriology and Virology of the University Hospital of Ghent, Ghent, Belgium. Isolates were subcultured on Sabouraud glucose agar (SGA) (Difco Laboratories, Detroit, Mich.) and were incubated for at least 72 h at 37°C.

### Clinical specimens.

Ear swabs (n = 26), sputum (n = 69), bronchoalveolar liquid (n = 29), serum (n = 4), tube culture (n = 1), nose swabs (n = 57), and blood (n = 2) were provided by the departments of Bacteriology and Virology, Otorhinolaryngology and Hematology of the University Hospital of Ghent.

### Filters, growth media, chemicals and reagents.

Nylon membrane filters (47-mm diameter, 0.45 µm pore size) (Gelman Sciences, Ann Arbor, Mich.) were used for filtration of the samples. Other filters, including nitrocellulose, polyamide, polysulfone, mixed cellulose esters and polytetrafluoroethylene filters were obtained from Millipore Corp. (Bedford, Mass.). Absorbent fiberglass pads were from Gelman Sciences.

Sabouraud glucose agar (SGA) was either supplemented with a combination of 2,500 µg/ml ticarcillin (2,344 µg/ml) -clavulanic acid (156 µg/ml) (Timentin®) (SmithKline Beecham Pharma, Genval, Belgium) to yield SGA-T, with a mixture of pyridoxine (10 ppm), thiamine (1.0 ppm), nicotinic acid (0.1 ppm), riboflavine (10 ppm), panthothenate (1.0 ppm), PABA (1.0 ppm), and inositol (10 ppm) (all from Sigma Chemical Co., St. Louis, Mo.), (SGA^{*}) or with both (SGA^{*}-T). Other growth media included potato glucose agar (PGA) (Difco), SGA supplemented with chloramphenicol (SGA+) (bioMérieux Vitek, Hazelwood, Mo.), SGA supplemented with gentamycin and vancomycin (Sigma), cornmeal agar with 0.5% Tween 80 (CMT) (Difco), and Czapek-Dox agar (CDA) (Atlas, 1993).

### Example 2 : Methods

### Screening for enzymatic activities.

The following 4-methylumbelliferyl (4-MU) derivatives were tested as enzyme substrates: acetate, N-acetyl-β-D-galactosaminide, α-L-arabinofuranoside, α-L-arabinopyranoside, α-D,D'-diacetylchitobioside, β-D-galactoside, β-D-glucoside, β-D-glucuronic acid, heptanoate, α-L-iduronide, β-D-lactoside, laurate, oleate, palmitate, phosphate, pyrophosphate and α-L-rhamnopyranoside. They were purchased from Sigma and Melford Laboratories (Ipswich, UK).

A 0.1 to 1 mg quantity of each substrate was dissolved in 1 ml of dimethylsulfoxide (Fluka, Buchs, Switserland), except 4-MU-palmitate and 4-MU-oleate, which were dissolved in dimethylformamide (Merck, Darmstadt, Germany). Stock solutions were diluted in 0.1 M phosphate buffer (pH 4.5) and sterilized by filtration over Nalgene disposable units (0.45 µm pore size, 250 ml) (Nalge Co., Rochester, N.Y.). The solutions were dispensed in test tubes and frozen at -20°C until use.

### Procedure.

Sputum and other mucous samples were first treated with 1% N-acetyl-L-cystein (Sigma) for 30 min at 37°C. In case of an insufficient filtration volume, they were diluted in 10 ml of physiological saline. The samples were divided in two equal parts and separately filtered over a 47-mm nylon membrane filter, with a pore size of 0.45 µm. After filtration, each of the membranes was placed on SGA-T^{*} medium and incubated at 45°C and 37°C, respectively, for at least 12 hours to yield microcolonies. As soon as colorless microcolonies became visible in daylight, the filters were removed and placed on an absorbent fiberglass pad, impregnated with 340 µl of a buffered solution containing 0.1% (w/v) 4-MU-α-L-arabinopyranoside, 0.1% (w/v) digitonin, acting as a membrane permeabilizer and 1 mM MgCl₂, acting possibly as a co-factor for the enzyme. After incubation for 30 min at 30°C, the filters were sprayed with 1.2 M sodium hydroxide and inspected under a 366 nm UV lamp. Blue fluorescent microcolonies indicated a positive reaction.

### Confirmation and reference method.

To confirm the identity of the fluorescent microcolonies, the membrane was reincubated at 45°C on the original SGA^{*}-T medium for at least 3 days and the mycelial growth was examined macroscopically and microscopically after lactophenol cotton-blue staining. As a reference, part of the original sample was also filtered over a second nylon membrane. Inoculation was performed at 37°C for 3 days on SGA+ and the typical fungal colonies were characterized by microscopy.

### Method evaluation.

The following equations were used to calculate the validity parameters of the method (TP = true positives; TN = true negatives; FP = false positives; FN = false negatives; n = total number of samples): sensitivity [TP x 100/(TP + FN)], specificity [TN x 100/(TN + FP)], positive predictive value [TP x 100/(TP + FP)], negative predictive value [TN x 100/(TN + FN)], and efficiency [(TP + TN) x 100/ n].

### Example 3 : Results

Enzyme activities in *A*. *fumigatus* and other fungal species were assessed from their ability to cleave a series of 4-MU glycosides and esters. In order to be useful for diagnostic purposes, the sensitivity of a given enzymatic reaction had to exceed 95%, i.e., less than 5% of strains of the target organism should be negative. Likewise, the specificity had to be ≥ 95%, i.e., the enzyme should occur in ≤ 5% of other fungal species.

Only the cleavage of 4-MU-α-L-arabinopyranoside satisfied both criteria (Table 1). The corresponding enzyme, tentatively called "arabinopyranosidase" occurred in 98% of *A*. *fumigatus* strains (n = 48), but was absent in other clinically important molds (n = 116) (Table 1). Among the yeasts, 5% of C. *albicans* strains tested (n = 129) showed a positive reaction with 4-MU-α-L-arabinopyranoside, whereas *C. glabrata* (n = 39), *C. krusei* (n = 25), C. *tropicalis* (n = 26) and *C. parapsilosis* (n = 14) were negative.

In physiological saline after ultrasonic lysis of cells or on SGA, fluorescence was weak. However, it was significantly more pronounced on a membrane filter. This was demonstrated at the cellular level by epifluorescence microscopy and after microcolony formation by inspection under a 366 nm UV lamp. Among the membrane filters compared, nylon proved clearly superior to nitrocellulose, mixed cellulose esters, polyamide, polysulfone and polytetrafluoroethylene in terms of visually scored intensity of fluorescence.

The rate of microcolony formation on the nylon membrane filter depended on the composition of the growth medium and the temperature, but was also influenced by the cellular stage of growth initially present in the sample (mycelium or spores) and the administration of antimycotics to the patient before sampling.

The growth promoting activity of the various media was compared in broth on the basis of turbidity measurements as a function of time. The enriched SGA^{*} yielded faster growth than SGA, CMT, PGA and CDA (results not shown). However, when using clinical samples, it was necessary to add an antibiotic to the medium to suppress bacterial growth. A combination of ticarcillin-clavulanic acid (Timentin®) has previously proven effective to this end (Bauters et al., 1999). To ensure that antibacterials had no negative effect on the proliferation of *A. fumigatus*, mycelial growth was monitored in broth for 44 h by turbidimetry. To this end, an amount of 10² conidia of three *A. fumigatus* strains were inoculated in 10 ml SGA^{*} and SGA^{*}-T broth and incubated (results not shown). No differences in growth rate were observed in both media. Growth at 45°C was superior to that at 37°C and entails additional selectivity towards bacteria and yeasts. However, some other molds were also able to proliferate at this temperature (Table 1). The positive contribution of the nylon membrane filter to the growth rate was demonstrated as follows. First, one hundred µl of a suspension of 10² conidia of *A*. *fumigatus* per ml (n = 5) was filtered over a nylon membrane filter (MF) and placed on SGA-T^{*} medium, supplemented with 1% 4-MU-α-L-arabinopyranoside. The same amount of conidia was inoculated by streaking (no MF) on a second plate with SGA-T^{*} medium, also supplemented with 1% 4-MU-α-L-arabinopyranoside. Both plates were incubated at 45°C and the different stages of growth were observed microscopically (Griffin, 1997). Hyphae started to grow and differentiate after an average time of 10 (MF) and 14 h (no MF), vesicles were observed after 14 and 17 h, while the end-point of the growth phase, i.e., the presence of phialides and conidia, was observed after 17 and 23 h, respectively. Microcolony formation was strongly affected by the developmental stage of the fungus in the sample. When starting from a sample containing mycelium, as in sputum or bronchoalveolar liquid, microcolonies appeared after approximately 11-14 hours. However, in case of spores the detection time was extended to at least 24 hours. Delayed growth was also observed in samples from patients treated with amphotericin B or itraconazole.

With the enzyme substrate added to the medium, the first fluorescent microcolonies on the membrane filter could be visualized after approximately 17 hours. However, in a two-step procedure where the membrane filter, after microcolony formation, is removed from the SGA^{*}-T and placed on an absorbent pad containing the substrate and 0.1% digitonin as a membrane permeabilizer, a further reduction of three hours in detection time was obtained.

The method has been applied to 188 clinical specimens, both positive and negative for *A. fumigatus.* The results with reference to a conventional plate isolation method with microscopic confirmation are listed in Table 2. Sensitivity and specificity were 96.9 and 100%, while the positive and negative predictive values were 100 and 95.8%, respectively. The overall efficiency of the method was 98.2%. No false positives were observed, whereas the false negative rate was 3.1%. Other *Aspergillus* spp. (*A*. *flavus*, n = 8; *A*. *niger,* n = 9; *A*. *nidulans,* n = 2), were isolated at 37°C but not at 45°C and gave no reaction with 4-MU-arabinopyranoside.

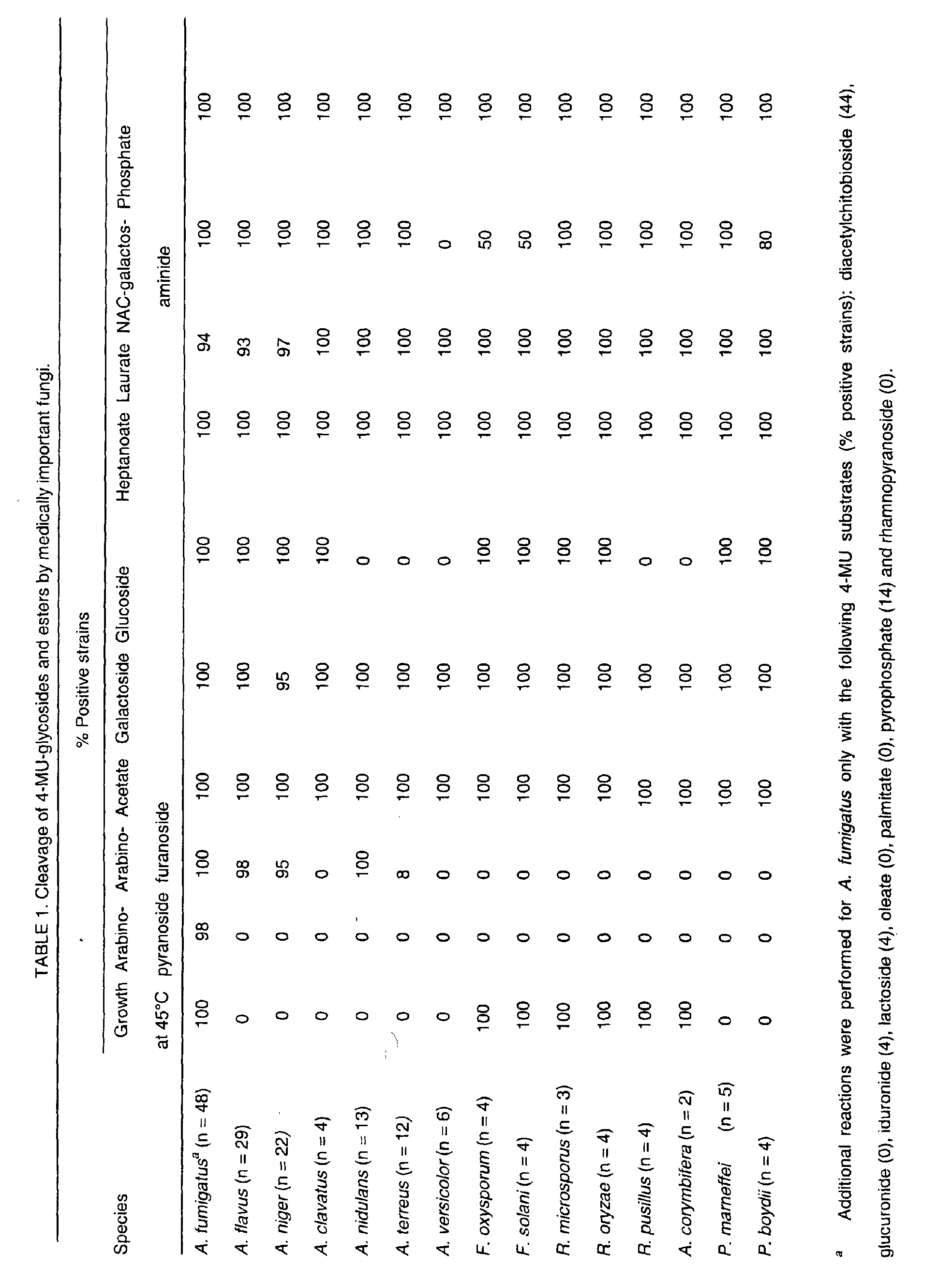

**TABLE 2.**

| Frequency table representing number of identified species comparing the arabinosidase based method and conventional identification methods. | | |
|---|---|---|
| | Conventional method | |
| Two-step method | *A. fumigatus* | Negative |
| *A. fumigatus* | 94 | 0 |
| Negative | 3 | 69 |

### REFERENCES

Atlas, R. M. 1993. *In* Handbook of Microbiological Media. CRC Press, Boca Raton, Florida.

Bauters, T. G., R. Peleman, M. Moerman, H. Vermeersch, L. Noens, and H. J. Nelis. 1999. Membrane filtration test for rapid presumptive differentiation of four *Candida* species. *J. Clin. Microbiol.* 37:1498-1502.

Bronstein, I., E. Brooks and J. Voyta. 1989. 1,2-Dioxetanes: novel chemiluminescent enzyme substrates. Applications to immunoassays. *Journal of bioluminescence and chemiluminescence* **4**:99-111.

Ferguson W.J. Method, test media and chromogenic compounds for identifying and differentiating general coliforms and *Escherichia coli* bacteria. US Patent 5,358,854.

Fridkin, S. K., and Jarvis W. 1996. Epidemiology of nosocomial fungal infections. *Clin. Microbiol. Rev.* **9**:499-511.

Griffin, D. H. 1993. The physical environment and growth, p. 195-214. *In* D. H. Griffin, Fungal physiology, 2^{nd} ed., Wiley-Liss Publisher, New York, NY.

Koller E. 1989. Fluorogenic enzyme substrates target the market of the biological sciences. *Applied Fluorescence Technology* **1**:1-10.

Latgé, J. -P. 1999. *Aspergillus fumigatus* and aspergillosis. *Clin. Microbiol. Reviews.* **12**:310-350.

Manafi, M., W. Kneifel and S. Bascomb. 1991. Fluorogenic and chromogenic substrates used in bacterial diagnostics. *Microbiological Reviews* **55**: 335-348.

Paugam, A., J. Sarfati, R. Romieu, M. Viguier, J. Dupouy-Camet, and J. P. Latgé. 1998. Detection of *Aspergillus* galactomannan: comparison of an enzyme-linked immunoassay and a europium-linked time-resolved fluoroimmunoassay. *J. Clin. Microbiol.* **36**:3079-3080.

Richardson, M. D., and D. W. Warnock. 1997. *In,* M. D. Richardson, Fungal infections, 2^{nd} ed., Blackwell Science, Oxford, UK.

Richardson, M. D. 1998. *Aspergillus* and *Penicillium* species, p.281-312. *In* Topley and Wilson's, Microbiology and microbial infections, Medical Mycology, 9^{th} ed., Arnold, London, UK.

Van Burik, J. -A., D. Myerson, R. Schreckhise, and R. Bowden. 1998. Panfungal PCR assay for detection of fungal infection in human blood specimens. J. *Clin. Microbiol.* **36**:1169-1175.

Verweij, P. E., J. - P. Latgé, A. J. Rijs, W. J. Melchiers, B. E. De Pauw, J. A. Hoogkamp-Korstanje, and J. F. Meis. 1995. Comparison of antigen detection and PCR assay using bronchoalveolar lavage fluid for diagnosing invasive pulmonary aspergillosis in patients receiving treatment for hematological malignancies. *J. Clin. Microbiol. 33*:3150-3153.

## Claims

1. A method for detecting micro-organisms in a biological sample comprising testing for an arabinopyranoside substrate cleaving activity of said micro-organism.

2. The method according to claim 1 wherein said micro-organism is *Aspergillus fumigatus.*

3. The method according to claim 1 or 2 wherein said testing of arabinopyranoside substrate cleaving activity comprises the use of a fluorogenic, chromogenic or chemiluminogenic substrate.

4. The method according to any of claims 1 to 3 wherein said arabinopyranoside substrate is 4-methylumbelliferyl-α-L-arabinopyranoside.

5. The method according to any of claims 1 to 4 comprising at least the following steps:
a) concentrating the micro-organisms in said biological sample on a membrane filter,
b) incubating the membrane filter of step a) on a growth medium to form microcolonies,
c) removing said membrane filter from the growth medium,
d) contacting said membrane filter with a solution of 4-methylumbelliferyl-α-L-arabinopyranoside possibly supplemented with a membrane permeabilizer,
e) possibly amplifying the signal by spraying said membrane filter with sodium hydroxide or another light amplifying material,
f) identifying the presence of light-emitting microcolonies on said membrane filter.

6. The method according to claim 5 wherein the membrane filter is a nylon membrane filter.

7. A growth medium for use in a method as defined in claim 5 or 6, said growth medium being Sabouraud glucose agar further comprising ticarcillin, clavulanic acid and a mixture of pyridoxine, thiamine, nicotinic acid, riboflavine, panthothenate, para-aminobenzoic acid (PABA) and inositol.

8. The method according to any of claims 5 to 7 wherein the incubation step b) is performed at 45°C.

9. A kit for diagnosing the presence of a micro-organism in a biological sample comprising an arabinopyranoside substrate.

10. A kit according to claim 9 comprising at least the following components:
a) a membrane filter,
b) a growth medium for the micro-organism(s) to be detected, and
c) a fluorogenic, chromogenic or chemiluminogenic arabinopyranoside substrate for testing the arabinopyranoside substrate cleaving activity.

11. A kit according to claim 10 wherein the membrane filter a) is a nylon membrane filter.

12. A kit according to claim 9 or 10 wherein b) is a growth medium as defined in claim 7.

13. A kit according to any of claims 9 to 11 further comprising an inducer for the arabinopyranosidase-substrate cleaving activity.

14. A kit according to any of claims 9 to 12 further comprising a membrane permeabilizer.
